# EUROPEAN PATENT APPLICATION

(11) **EP 3 932 335 A1**
(43) Date of publication of application: **05.01.2022**
(21) Application number: 20183429.8
(22) Date of filing: 01.07.2020
(51) Int. Cl.: A61B 17/12, A61B 17/00, A61B 17/11

(54) **A DEVICE FOR TREATMENT OF ANASTOMOTIC LEAK**

(71) Applicant: Bansal, Aakanksha, 51065 Köln (DE)
(72) Inventor: Dr. BANSAL, Aakanksha, 51065 Köln (DE); DERYNCK, Zeger, 3210 Lubbeek (BE)
(74) Representative: Grünecker Patent- und Rechtsanwälte PartG mbB

(57) **Abstract**

The present invention relates to a device for treatment of an anastomotic leak comprising a catheter (12), wherein an end of the catheter (12) is configured to be positioned at a target site of the anastomotic leak, having predetermined material, and a dispensing means (18) configured to provide a biodegradable and/or self-absorbable biomaterial (24) in the target site such that the predetermined material is self-absorbed by the biomaterial.

## Description

### Field of Invention

The present invention relates to a device for treatment of an anastomotic leak, particularly where post-operative fistula formation occurs after a gastrointestinal surgical procedure.

### Background Art

The gastrointestinal (GI) tract is a series of digestion-related hollow organs connected to each other, forming a continuous passageway from the mouth to the anus. The organs include the mouth, esophagus, stomach, small intestine, large intestine, and anus. The GI tract may be divided into the upper and lower GI tracts.

Gastrointestinal surgery is commonly conducted on a selected part of the GI tract for treatment of digestive disorders or diseases of the GI tract, such as esophageal cancer. An example of a gastrointestinal surgery is esophagectomy, wherein a portion or all of the esophagus is surgically removed and the stomach is joined with the remaining esophagus part or a replacement hollow structure, such as a section obtained from the patient's colon.

In spite of the advances in medical surgical procedures, the mortality rate for gastrointestinal surgery can be greatly affected by the management of post-operative complications, which can be life-threatening. One common post-operative complication can be anastomotic leakage. In the case of the esophagectomy procedure, anastomotic leakage can occur when there is fistula formation at the surgical join site, resulting in the entry of digestive fluids to the thorax, thereby causing inflammation. The body's immune system then can mount a natural response to the inflammation and encapsulate the fistula, thereby creating an abscess cavity containing the fluids.

Various methods of treatment for anastomotic leakage depend on the removal of accumulated fluid in the abscess cavity. As the fluid is removed from the cavity and the cavity can be kept free of fluids, the abscess cavity could shrink and the fistula could eventually close.

A known anastomotic leakage treatment method is the Endoluminal Vacuum Therapy (E-VAC) system. This vacuum-assisted therapy method utilizes an open-pore polyurethane sponge (Eso-SPONGE^{®}) connected to a vacuum system via a drainage tube and inserted into the abscess cavity to drain out the fluids from the abscess cavity.

In using the E-VAC system, a flexible endoscope is first inserted via the patient's mouth to investigate and measure the abscess cavity. An overtube is then inserted using the endoscope as a guide to reach the site of the abscess cavity. A suitably tailored Eso-SPONGE^{®} is then, for example, covered by sterile hydrogel and inserted into the open end of the overtube. A pusher can be used to push the sponge to the end of the overtube to reach the site of the abscess cavity. The sponge unfolds into the cavity when the overtube is withdrawn from the cavity and out of the patient. The positioning of the sponge may require further adjustment and correction by means of endoscopic grasping forceps. In order for the drainage tube to be transnasally channeled instead of via the patient's mouth, a separate stomach tube may be required to be inserted through the nose and brought out of the mouth for connection to the drain tube of the Eso-SPONGE^{®}.

### Summary of Invention

### Technical problem

However, the E-VAC system may need to be managed every 48-72 hours. In particular, the used sponge would have to be removed from the cavity and replaced with a new Eso-SPONGE^{®} via a new endoscopic procedure to continue with the treatment of anastomotic leak. This is because sponges left in the cavity far too long could become clogged and could become increasingly difficult to remove, thus no longer providing effective treatment to help the patient. The average number of sponge replacement can range between about 2 to as high as 10 per patient. Each sponge replacement could require the removal and insertion of the Eso-SPONGE^{®} via endoscopy, thereby requiring multiple and frequent endoscopic interventions, which, however, may not be desirable from a medical perspective and also from a patient comfort level standpoint. Performing multiple endoscopic procedure can also become expensive.

Against this background, it is the object of the invention to provide an improved medical device suited for treatment of anastomotic leakage, which can be minimally invasive and requires fewer endoscopic interventions than the conventional approach.

### Solution to Problem

According to the present invention, this object is achieved by the features of independent claim 1.

According to an aspect of the invention, a device for treatment of an anastomotic leak comprises a catheter, wherein an end of the catheter is configured to be positioned at a target site of the anastomotic leak, having predetermined material; and a dispensing means, configured to provide a biodegradable and/or self-absorbable biomaterial in the target site such that the predetermined material is self-absorbed by the biomaterial.

The device may be inserted and guided to the target site using conventional endoscopic technique. For example, the device may be guided using known endoscopic procedure, via mouth, nasal, or anal passage of a patient. One end, preferably, the distal end of the catheter can be guided, via a guide tube and the likes, to reach the target site of the anastomotic leak where predetermined material such as wound secretions, organic debris, for example, digestive fluids, clots, debris may be present. The end of the device having the catheter may be conveniently guided and positioned at the target site of the anastomotic leak (for e.g., fistula, abscess cavity) with one endoscopic procedure, thereby avoiding the need for a separate procedure, for example, to adjust the position of the catheter using endoscopic forceps after insertion of the catheter as in the conventional approach.

The dispensing means is adapted to provide the biodegradable and/or self-absorbable biomaterial in the target site of the anastomotic leak. The biomaterial can be self-absorbable such that the predetermined material in the target site can be self-absorbed by the biomaterial. The biomaterial is degradable and can remain in the target site during the treatment of the anastomotic leakage and also during healing of the inflammation around the target site. As the biomaterial is biodegradable and/or self-absorbable, there is no need for removal of the biomaterial from the patient during the treatment and also after the treatment is complete. The biomaterial may shrink as the abscess cavity shrink and may be completely biodegraded as the abscess cavity completely heals and closes. Accordingly, the need for multiple and/or expensive endoscopic interventions in the conventional approach can be avoided and the patient's comfort level can be improved.

The device according to another aspect of the invention may further comprise a securing means coupled to the end of the catheter. The securing means may be configured to be movable to an open position for securing the end of the catheter to the target site.

With the securing means which is coupled to the catheter, the catheter can be secured at or around the target site. In particular, the securing means may be movable to an open or expanded position and, for example, to a closed or retracted position. In the open position, the securing means may be configured to secure the catheter at or around the target site. Thus, the open position of the securing means can act, for example, like an anchor to securely hold the catheter at the target site. In some embodiments, the securing means can thus facilitate positioning of the catheter at the target site so that the securing means can continue to hold the catheter in place when in use within the patient and/or during the treatment. The need for a separate procedure to hold or position the catheter via endoscopic forceps can therefore be avoided.

For example, the target site may include an opening through which the end of the catheter can be guided into the target site and the biomaterial can be provided in the target site. The securing means may act like an anchor to secure the end of the catheter to the target site at or around the opening of the target site.

Advantageously, the securing means coupled to the end of the catheter may be in the closed or retracted position during insertion of the catheter into the patient so as not to interfere with the insertion process, and then can be moved to the open position when the end of the catheter is positioned at or around the target site for securing the end of the catheter to the target site.

In the device according to another aspect of the invention, at least a part of the securing means may be within the catheter. With this configuration, at least the part of the securing means can remain or be retracted within the catheter during deployment of the device into the patient, such that the securing means do not unnecessarily interfere with the insertion process of the catheter. For example, because at least the part of the securing means is within the catheter, the securing means do not have to be in the open position during the deployment of the catheter into the patient but can be moved to the open position to secure the catheter to the target site once the catheter is positioned at the target site. The securing means can be bendably or foldably secured within the catheter, in particular it its retracted position so that the retracted securing means remain protected within the catheter and does not interfere with the movement of the catheter within the patient, for example, along the nasal passage. In some embodiments, the at least the part of the securing means, for example, the proximal end, can be fixed to a part, in particular to the end of the catheter. In some embodiments, said part of the securing means, in its open position, can extend from the end of the catheter so that the distal end of the securing means can be secured to the target site.

As the securing means is coupled with or within the catheter, with one endoscopic procedure, both the catheter and the securing means can be inserted into the patient, thereby it is possible to avoid insertion of a separate means and/or any additional components, via separate endoscopic procedure or like, to secure the catheter to the target site. Further, because the securing means is coupled to the catheter, this feature can also function as a joint to securely hold the catheter at or around the target site and avoid unnecessary or inadvertent movement of the catheter away from the target site.

The device according to another aspect of the invention may further comprise an absorbing means configured to absorb the predetermined material before and/or after the providing of the biomaterial in the target site.

With the absorbing means, the predetermined material such as wound secretions or organic debris, for example, digestive fluids, clots, debris at the fistula formation can be removed from the target site at any desired time, i.e. before, after, or before and after, providing the biomaterial to the target site. It can be advantageous to remove the predetermined material, for e.g., by absorbtion, before providing the biomaterial so that the space in the target site is sufficiently clear enough to accommodate the biomaterial in the target site. Also, this way, the absorbing means can act as a pre-remover or a pre-absorber for absorbing parts of the predetermined material before the biomaterial is dispensed in the target site and the biomaterial can further absorb the rest of the predetermined material from the target site.

In some embodiments, it can be advantageous to perform the absorbtion after providing the biomaterial to absorb parts of the predetermined material which, for example, are not accessible by the biomaterial.

In the device according to another aspect of the invention, at least a part of the absorbing means may be arranged within the catheter. Accordingly, a part of the absorbing means can be arranged within the catheter before or during deployment of the device into the patient, such that the absorbing means do not unnecessarily interfere with the insertion process of the catheter. Another advantage is that the catheter can act as an outer frame for easy insertion and/or replacement of the absorbing means while the catheter is in-situ at the target site without having to withdraw and remove the catheter from the target site.

In the device according to another aspect of the invention, the absorbing means may comprise a tube provided with vacuum suction. Vacuum suction is a conventionally known technique and can be advantageously used in the device to remove parts of the predetermined material from the target site and out of the patient as needed, without requiring additional endoscopic interventions. In the device according to another aspect of the invention, at least a part of the dispensing means may be arranged within the catheter. Accordingly, a part of the dispensing means can be arranged within the catheter before or during deployment of the device into the patient, such that the dispensing means do not unnecessarily interfere with the insertion process of the catheter. Another advantage is that the catheter can act as an outer frame for easy insertion and/or replacement of the dispensing means while the catheter is in-situ at the target site without having to withdraw and remove the catheter from the target site.

In the device according to another aspect of the invention, the dispensing means may comprise a dispensing tube connected to a biomaterial supply device. Accordingly, an advantage of this is that the dispensing tube can be easily convey the selected biomaterial to the target site as needed, without requiring additional endoscopic interventions.

In the device according to another aspect of the invention, the catheter may comprise biocompatible material that is resistant to fragmentation. An advantage of this is that the catheter does not fall apart while being deployed, in use within the patient, or during removal from the target site.

In the device according to another aspect of the invention, the securing means may comprise biocompatible material that is resistant to fragmentation. An advantage of this is that the securing means continues to hold the catheter in place when in use within the patient such that the catheter can continue to provide the required treatment to the patient. Also, the material of securing means may be unreactive to the body tissue and may not get embedded or stick to the tissue so that it can be easily removed even after staying couple of days inside the body.

In the device according to another aspect of the invention, the biomaterial can be any one of a natural biodegradable polymeric biomaterial and synthetic biodegradable polymer, or a combination, or a derivative thereof. In some embodiments, the natural biodegradable polymeric biomaterial can be selected from any one of gelatin, collagen, fibrin, hyaluronic acid, chitin/chitosan, starch, silk, alginate and polyhydroxyalkanoate, and the synthetic biodegradable polymer can be selected from any one of saturated aliphatic polyester such as poly(glycolic acid) (PGA), PLA, and PLGA copolymer, polyanhydride ,polyurethane, polyphosphazene.

As the naturally and/or synthetically biodegradable polymers are used, their self-absorbable and biocompatible characteristics renders them useful in the treatment of the anastomotic leakage, thereby avoiding there need for removal of the biomaterial from the patient during the treatment and also after the treatment is complete. In particular, the biomaterial can be injected for easy delivery to the target site, the biomaterial is biodegradable and hence does not require subsequent removal by endoscopic procedures, and may be biocompatible hence can be suited for use within the human body. Accordingly, the need for multiple and/or expensive endoscopic interventions in the conventional approach can be avoided and the patient's comfort level can be improved. That is, since a biomaterial is used instead of the sponges of the conventional approach, multiple endoscopic procedures can be avoided.

In another aspect of the invention, the biomaterial may take the shape of the target site. Accordingly, the biomaterial could access the entire target site and self-absorb the predetermined material in the target site, thereby improving efficiency of the treatment.

In the device according to another aspect of the invention, the biomaterial may be detectable with an imaging technique. Accordingly, the user of the device could easily track the biomaterial so as to access the healing state of the abscess cavity, the time remaining for the healing, the amount of biomaterial remaining and judge whether replenishing may be necessary, etc. For example, the healing state of the abscess cavity can be easily monitored by detecting the biomaterial and its remaining amount via imaging techniques, which are simpler and more comfortable to the patient than endoscopic procedures. In the device according to another aspect of the invention, the biomaterial may be chargeable with antibiotics. With this feature, the healing of the abscess cavity may be boosted by the application of antibiotics via the biomaterial. The antibiotics can also act against bacterial and viral growth in the target site.

In another aspect of the invention, the biomaterial may be porous so as to allow the predetermined material to pass therethrough the absorbing means. With this feature, the biomaterial can facilitate removal of the predetermined material via the self-absorption and also through its pores via the absorbing means. That is, the biomaterial can stay in place at the target site and facilitate the removal of the predetermined material while the predetermined material is removed from the target site by the absorbing means such as, for example, vaccum suction.

In another aspect of the invention, the biomaterial may be provided in a flowable form and configured to coagulate to a non-flowable form after a predetermined time. An advantage of this is that the biomaterial in flowable form can fill up the target site and then remain at the site after coagulating to a non-flowable form after a predetermined time according to the chosen biomaterial. This form can also ease the handling of the biomaterial and dispensing of the biomaterial at the target site. For example, the biomaterial can be injected in liquid or semi-solid form and, thus the biomaterial could take the shape of the target site (for e.g., abscess cavity, fistula) thereby tightly sealing the target site leaving no space for any further body fluids to accumulate in the target site. For example, a faster coagulation rate of the biomaterial may ensure that the dispensed biomaterial does not get much time to seep out of the target site and quickly adapt to the shape of the target site.

### Brief Description of the Figures

Aspects of the invention will now be described by way of example only, with reference to the accompanying drawings.
Figure 1 shows a simplified cross-section view of a device for treatment of an anastomotic leak with a securing means in a retracted position, according to one aspect of the invention.
Figure 2 shows a simplified cross-section view of the device for treatment of an anastomotic leak with the securing means in an open position, according to another aspect of the invention.
Figure 3 shows a simplified cross-section view of the device for treatment of an anastomotic leak further comprising a portion of an absorbing means within the catheter, according to another aspect of the invention.
Figure 4 shows a simplified cross-section view of the device for treatment of an anastomotic leak further comprising a portion of the absorbing means and a portion of a dispensing means within the catheter, according to another aspect of the invention.
Figure 5 illustrate a method of treating an anastomotic leak using the device in accordance with an aspect of the invention.
Figure 6 shows a simplified cross-section view of the device for treatment of an anastomotic leak further comprising a portion of an absorbing cum dispensing means within the catheter, according to another aspect of the invention.

### Detailed Description

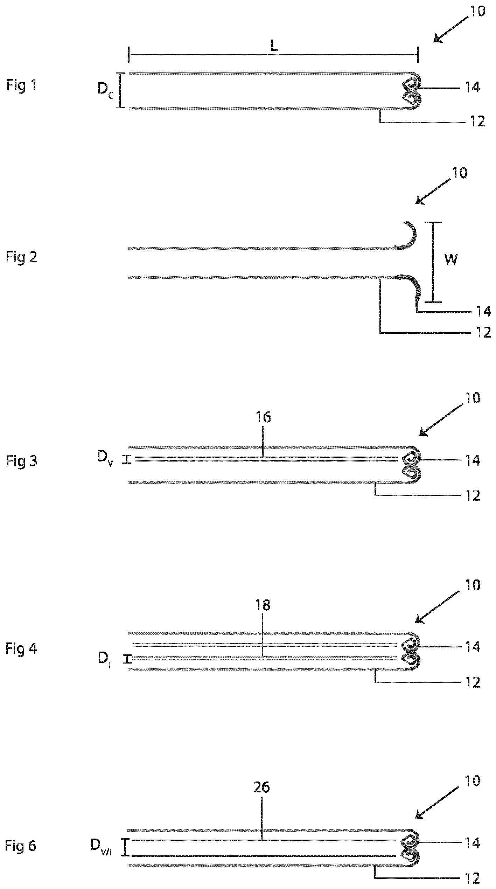

Referring to Figs. 1 to 4, the configuration of a device for treatment of an anastomotic leak according to an embodiment of the present invention will be described below.

Fig.1 illustrates the device for treatment of an anastomotic leak according to an aspect of the present invention. As shown in Figs. 1 to 4, the device 10 for treatment of the anastomotic leak includes a catheter 12, a securing means 14, an absorbing means 16, and a dispensing means 18.

The catheter 12, which acts as a guiding catheter, includes two open ends. As is conventionally known, catheters are medical devices comprising tubular elements which may be inserted into a patient for various medical purposes, such as to provide drainage from, or to administer fluids to patients, or to provide access to certain parts of the patient's body such as stomach, intestine, nasal passage and the likes. That is, the catheter can form a flow passage from/to the outside of the patient to/from the certain parts of the patient's body.

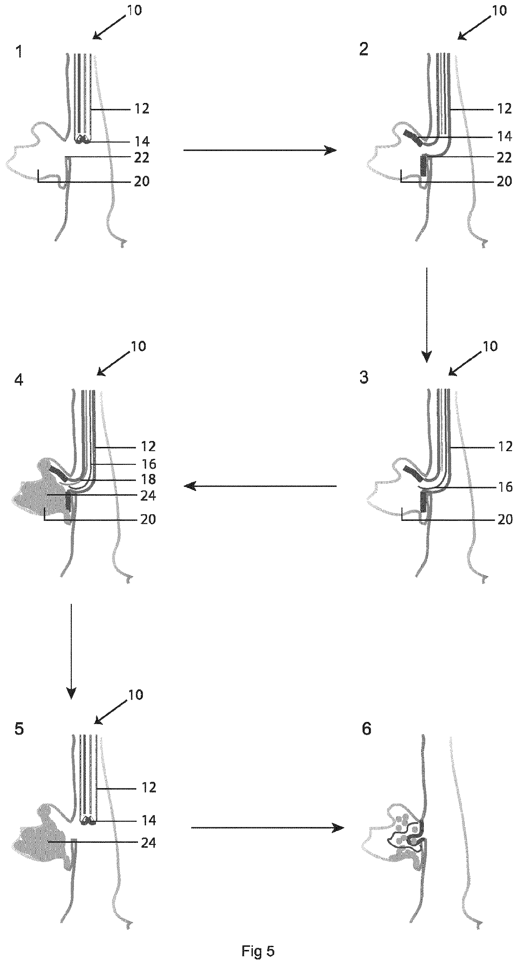

In Fig. 1, the guiding catheter 12 is adapted to be suitable for deployment through a selected passage in a patient, such as a part of the gastrointestinal tract as the case may be. For example, the catheter 12 may be deployed through the mouth and/or the nasal passage of the patient into an abscess cavity 20 (shown in Fig. 5) in the upper gastrointestinal tract. The abscess cavity 20, and/or the fistula is an example of the target site. The shape of the catheter 12 is not limited but can be any one of a tubular shape with square or hexagonal or the likes in cross-section or a cylindrical shape.

In one embodiment, the catheter 12 has a cylindrical shape. The diameter D_{c} of the guiding catheter 12 may be between 1.33 mm and 8 mm. Preferably, the diameter D_{c} of the catheter 12 is 5 mm. The diameter D_{c} of the catheter 12, however, is not limited to 1.33-8 mm. The diameter D_{c} of the guiding catheter 12 be such that the catheter 12 is deployable in the chosen passage of the human body.

In one embodiment, the length L of the guiding catheter 12 may be between 50 cm and 130 cm. Preferably, the length L of the catheter 12 is at least about 110 cm. In other embodiments, the length of the guiding catheter 12 is such that when one end of the catheter is positioned at the abscess cavity 20, the other end of the catheter may either lead out of the patient or be connectable to another tube/device for connection to an appropriate medical device or equipment, such as an intravenous therapy fluid bag, a drainage bag or a vacuum pump, as non-limiting examples.

As shown in Fig. 1 to Fig. 5, the securing means 14 is configured to secure the catheter 12 at or around the abscess cavity 20. In particular, the securing means 14 acts as an anchor to secure the device including the catheter to the abscess cavity 20 of the anastomotic leak throughout the treatment procedure(s) (described below).

As shown Fig. 1 and 2, the securing means 14 is coupled to the end of the guiding catheter 12. In particular, the securing means 14 is coupled in the vicinity of the open end of the catheter 12, such as, for example, the open end of the catheter 12, a predetermined distance within the interior or exterior surface of catheter 12 from the open end of the catheter 12.

Preferably, the securing means 14 may be moveable between a retracted position within the catheter 12 as shown in Fig.1 and an open position or an extended position as shown in Fig 2. In the open or extended position, the securing means 14 can attach the catheter 12 to an opening 22 of the abscess cavity 20 (references 20 and/or 22 can be regarded as the target site) and can, thereby secure the catheter 12 at the opening 22 of the abscess cavity 20 during the treatment procedure(s).

For example, the open end of the catheter 12 may be positioned at the opening 22 of the abscess cavity 20 with the securing means 14 in the retracted position as shown in Fig.1 and Fig. 5(1). The catheter 12, and thereby the device 10, can be secured in place at the abscess cavity 20 by moving the securing means 14 to the open position as shown in Fig.2 and Fig. 5(2) for conducting the treatment procedure(s) at the abscess cavity 20.

In a preferable embodiment, the securing means 14 has one or more foldable elements coupled to the catheter 12. The foldable element may be a 2-dimensional or a 3-dimensional element. The foldable element(s) is configured such that, in the retracted position, the foldable element(s) is wrapped, for example spirally, within the catheter 12 (shown, for example, in Figs. 1, 3, 4, 5(1) and 5(5)). In an alternative embodiment, the foldable element can also retract within the catheter 12 by extending along the length of the catheter 12. In the open position, the foldable element(s) can extend from the end of the catheter 12 (shown, for example, in Figs. 2 and 5(2)-5(4)). The foldable element can preferably be manipulated by an external operating means (described below) so as to achieve a specific curvature and to move from/to the retracted position to/from the open position. The invention is not limited to the shape of the foldable element. For example, the foldable element can be any one of a pair of hook element, a donut shaped ring element, a ring of overlapping flaps, and the likes.

The size of the abscess cavity 20, in particular, the width of the opening 22 is not limited but can preferably range from 5 - 8 mm. The width W of the securing means 14 in the open position can preferably be more than the width of the opening 22, i.e., about 8 - 10 mm, for secured positioning of the catheter 12. In a preferred embodiment, the dimensions of the securing means 14 can be chosen depending on the necessity, the size of the abscess cavity 20 and the width of the opening 22 as long as the securing means 14, in the open position, has the width W sufficiently wide to be anchored in the abscess cavity 20.

The abscess cavity 20 of the anastomotic leak can have predetermined material such as wound secretions, organic debris, for example, digestive fluids, clots, debris and the likes. As shown in Fig. 3, the device 10 is further provided with an absorbing means 16. The absorbing means 16 or a part thereof, such as an absorbing or vacuum tube, can be located within the catheter 12. The absorbing means 16 may comprise the vacuum tube connected to an external vacuum pump for facilitating suction of the predetermined material from the abscess cavity 20. The vacuum pressure is set to be sufficient to suck out the predetermined material collected in the abscess cavity 20. In use, the vacuum tube is located within the catheter 12 and can thus be protected from exposure to bodily fluids during insertion of the catheter 12 into the patient, such as nasal or mouth passage, and/or the tract. While the vacuum tube needs to fit within the catheter 12, the diameter Dᵥ of the vacuum tube also needs to be sufficiently large enough to prevent clogging of the vacuum tube by fluids, clots and dead cells removed from the abscess cavity 20. The diameter Dᵥ of the vacuum tube is preferably about 2.4 mm - 5 mm

As shown in Fig. 4, the device 10 is further provided with a dispensing means 18. The dispensing means 18 or a part thereof, such as a dispensing tube, can be located within the catheter 12. The dispensing means 18 may comprise the dispensing tube connected to an external supply source containing the substance for dispensing. The diameter Dₗ of the dispensing tube is preferably about 2.4 mm - 5 mm.

The substance for dispensing may be biomaterial 24 for filling the abscess cavity or therapeutic drugs or a combination of both. The biomaterial 24 is preferably biocompatible and preferably may not aggravate the inflammatory response at the abscess cavity 20. The biomaterial 24 is preferably provided in a flowable form such as in liquid or slurry form. The biomaterial 24 can preferably coagulate to a non-flowable form within a predetermined time in the abscess cavity 20. The predetermined time period is dependent on the choice of biomaterial. For example, in the case of the biomaterial 24 being gelatin, the coagulation could occur within 5 to 10 minutes (for e.g., 9.5 minutes for gelatin foam form, 6.2 minutes for gelatin in powder form).

The biomaterial 24 is preferably self-absorbable such that it is capable of absorbing the predetermined material fluids in the abscess cavity 20. The biomaterial 24 may also be biodegradable such that it can be broken down by living organisms within the human body, or bio-absorbable wherein the biomaterial may be absorbed by living tissue. In either cases, the biomaterial does not require removal from the abscess cavity 20 by endoscopic procedures or the likes. The abscess cavity or fistula typically takes about 4-6 weeks to heal and shrink, thus, the biodegradable biomaterial 24 may preferably degrade within about the same time period or earlier. In the case of the bio-absorbable biomaterial, the period for absorption into the body may be within the same time period or earlier.

The biomaterial 24 may preferably be able to take the shape of the abscess cavity 20 so as to substantially completely fill said cavity 20 (see Fig. 5 (4) and Fig. 5(5)). The biomaterial 24 may preferably not facilitate the further accumulation of the liquid in the abscess cavity 20 and may not support bacterial and viral growth in said cavity 20. The biomaterial 24 may preferably be chargeable with antibiotics or other therapeutic drugs. The biomaterial 24 may preferably be trackable by conventional imaging techniques such as fluoroscopy, magnetic resonance imaging (MRI), computed tomography (CT), ultrasound and the likes. The viscosity of the dispensed biomaterial may preferably be different from the viscosity of the predetermined material in the abscess cavity 20. The biomaterial 24 may preferably be porous so as to allow the predetermined material to pass through when vacuum suction is applied. The absorbing means 16 is configured, for example, using a selective filter and the likes, such that the biomaterial 24 is not absorbed by the absorbing means.

The biomaterial 24 can be any one of a natural biodegradable polymeric biomaterial and a synthetic biodegradable polymer, or a combination, or a derivative thereof. The natural biodegradable polymeric biomaterial is selected from any one of gelatin, collagen, fibrin, hyaluronic acid, chitin/chitosan, starch, silk, alginate and polyhydroxyalkanoate, and the synthetic biodegradable polymer is selected from any one of saturated aliphatic polyester such as poly(glycolic acid) (PGA), PLA, and PLGA copolymer, polyanhydride, polyurethane, polyphosphazene. An example of a biomaterial according to an aspect of the invention is gelatin. Other examples of the biomaterial may include collagen, hydrogels, alginate and the like.

A handle (not shown) for manipulating the device 10 can be provided at the user end or proximal end, that is away from the abscess cavity 20. The handle can include one or more operating means for guiding the catheter 12 in the gastrointestinal tract, for positioning the catheter 12 at the abscess cavity 20 and the likes. Similarly, the absorbing means 16 and the dispensing means 18 can be manipulated or controlled at the user end using the operating means. The operating means can also control the securing means 14 so as to move the securing means 14 between the open and the retracted position. Any conventional technique can be employed as the operating means.

Preferably, the operating means acts as an actuating means and/or a force transmitting means for positioning and functioning of the catheter 12, the securing means 14, the absorbing means 16 and the dispensing means 18. For example, one or more knobs, rotation or pressing of which, can navigate the catheter 12 in the tract and facilitate the positioning of the catheter 12.

Similarly, the securing means 14 can be controlled such that, when moving from or to the retracting/open position, a specific curvature of the foldable elements may be achieved by exerting tensile forces by means of the operating means, for example during securing of the catheter 12 at the abscess cavity 20. Draw cables or draw wires as the operating or force transmission means can be guided through the internal hollow outer catheter 12 to the proximal end region of the foldable elements. Preferably, the force application points of two such draw cables or draw wires are diametrically opposed. Thus a specific effect can be obtained of the curvature of the foldable elements and consequently of the catheter 12 exerting a tensile force by means of the operating means, when the catheter 12 with the securing means 14 is, for example, pushed through the tract and to the abscess cavity 20. The invention is not limited thereto, and any conventional technique to open and retract the foldable elements can be used.

Preferably, the catheter 12, the absorbing means 16 and the dispensing means 18 can be bendable so as to follow the shape of the passages in the human body and/or to be inserted into the opening 22 of the abscess cavity 20.

The catheter 12 and the securing means 14 may be made from material that is biocompatible, robust and resistant to fragmentation. As non-limiting examples, the catheter 12 and the securing means 14 may be made from biocompatible polymers, such as silicone rubber, nylon, polyurethane, polyethylene terephthalate (PET), latex, and thermoplastic materials such as polyurethanes, and thermoset elastomers such as silicone and hydrogels. The guiding catheter 12 and the securing means 14 may preferably not stick to the tissue or get cemented in the biomaterial. In a preferred embodiment, the guiding catheter 12 may have a suitable coating and design to prevent the catheter 12 and the securing means 14 from sticking to the tissue or getting cemented in the biomaterial. For example, the guiding catheter 12 may have a thin hydrophilic surface coating that when immersed in orcomes into contact with fluids, the surface coating swells to a smooth, slippery film which prevents the catheter 12 from sticking to the tissue or getting cemented in the biomaterial.

The vacuum tube and/or the dispending tube can be made from biocompatible material such as silicone rubber, nylon, polyurethane, polyethylene terephthalate (PET), latex, thermoplastic elastomers and so on. The vacuum tube may have to remain in the patient as inner tube of the catheter 12 for a desired period of use, which can typically be 1-2 weeks after the catheter 12 is deployed to the abscess cavity 20. Preferably the dispensing tube is resistant to the substance for dispensing and can maintain its functional quality within the desired period of use. The catheter 12 having therein the vacuum tube and/or dispensing tube can preferably be removed after dispensing the biomaterial in the abscess cavity 20.

Referring to Fig. 5, a method for treating an anastomotic leak in accordance with another aspect of the invention will be described. Fig. 5 describes a process of deploying and retrieving the device.

Fig. 5(1) shows the deploying of the device 10 via a conventional endoscopic procedure through the nasal or mouth passage of the patient to reach the vicinity of the site of fistula, i.e., the abscess cavity 20. In another aspect of the invention, the device 10 may be inserted into the patient via other passages, such as other parts of the gastrointestinal tract. The device 10 can include only the catheter 12 during this deployment. Preferably, the device 10 includes the catheter 12 and at least the securing means 14, parts of the vacuum tube 16 and parts of the dispending tube 18 within the catheter 12 during this deploying step. Thus, the end of the device 10 having the catheter 12 with said components can be conveniently guided and positioned at the abscess cavity 20 of the anastomotic leak (for e.g., fistula) with one endoscopic procedure, thereby avoiding the need for a separate procedure, for example, to adjust the position of the catheter using endoscopic forceps after insertion of the catheter as in the conventional approach.

As mentioned above, the vacuum tube 16 and/or the dispensing tube 18 can be arranged within the catheter 12 before or during deployment of the device 10 into the patient, such that the vacuum tube 16 and/or the dispensing tube 18 do not unnecessarily interfere with the insertion process of the catheter 12. Another advantage is that the catheter 12 can act as an outer frame for easy insertion and/or replacement of the vacuum tube 16 and/or the dispensing tube 18 while the catheter 12 is in-situ at the abscess cavity 20 without having to withdraw and remove the catheter 12 from the abscess cavity 20.

In the deploying step, the securing means 14 is in the retracted position within the catheter 12. Thus, the securing means 14 do not unnecessarily interfere with the deployment process of the catheter 12. For example, the securing means 14 is retracted within the catheter 12 during deployment, but can be moved to the open position to secure the catheter 12 to the abscess cavity 20 once the catheter is positioned at the abscess cavity 20. The securing means 14 can be bendably or foldably secured within the catheter 12, in particular it its retracted position so that the retracted securing means remain protected within the catheter 12, thereby avoiding exposure to the body tissue and does not interfere with the movement of the catheter within the patient, for example, along the nasal passage.

Referring to Fig. 5(2), the open end of the catheter 12 is positioned at the opening 22 of the abscess cavity 20. The catheter 12 is manipulated, either by the endoscopic procedure or by the operating means, by bending its open end so as to insert the open end of the catheter 12 into the opening 22. The securing means 14 is moved from the retracted position to the open position, thereby to secure the open end of the catheter 12 to the opening 22. The securing means 14 may remain in the open position during the treatment process, so as to securely hold the catheter 12 at the opening 22. In some embodiments, the securing means 14, in its open position, extends from the open end of the catheter 12 so that the distal end of the securing means 14 can be secured to the abscess cavity 20.

As the securing means 14 is coupled with or within the catheter 12, with one endoscopic procedure, both the catheter 12 and the securing means 14 is inserted into the patient, thereby it is possible to avoid insertion of a separate means and/or any additional components, via separate endoscopic procedure or like, to secure the catheter to the abscess cavity 20. Further, because the securing means 14 is coupled to the catheter 12, this coupling can also function as a joint to securely hold the catheter 12 in or at or around the abscess cavity 20 and avoid unnecessary or inadvertent movement of the catheter 12 away from the abscess cavity 20. Preferably, in this embodiment, the catheter 12 is provided with the absorbing means 16 (vacuum tube) and the dispensing means 18 (dispensing tube) during the deployment of the device so that in one endoscopic procedure all the essential elements are securely positioned at the abscess cavity 20, thereby avoiding multiple endoscopic procedures or multiple deployment procedures. Referring to Fig. 5(3), the absorbing means 16 is used to absorb the predetermined materials such as bodily fluids, wounds, debris, clots etc., within the abscess cavity 20. For example, the vacuum tube 16 connected to the external vacuum pump is bendably inserted into the opening 22 and can absorb the collected fluids, clots, and debris from the abscess cavity 20 so as to clean said cavity 20. It can be advantageous to remove the predetermined material, for e.g., by absorption, before providing the biomaterial 24 so that the space in the abscess cavity 20 is sufficiently clear enough to accommodate the biomaterial 24 in said cavity 20. Also, this way, the absorbing means 16 can act as a pre-remover or a pre-absorber for absorbing parts of the predetermined material before the biomaterial 24 is dispensed and the biomaterial can further absorb the rest of the predetermined material from the abscess cavity 20.

As illustrated in Fig, 5(4), after the cavity 20 is cleaned by the vacuum suction, the biomaterial 24 is dispensed into the abscess cavity 20 to substantially fill up said cavity 20.

Although not shown in Fig. 5, it is also possible to dispense the biomaterial 24 into the abscess cavity 20 prior to cleaning said cavity 20 by vacuum suction. The biomaterial 24 may have self-absorption property, such that the biomaterial 24 is capable of self-absorbing the fluids within the abscess cavity 20 when the material 24 is dispensed into the abscess cavity 20. The vacuum tube 16 may then be deployed to convey the self-absorbed fluids away from the biomaterial and out from the patient. That is, in some embodiments, it can be advantageous to perform the absorption after providing the biomaterial 24 to absorb parts of the predetermined material which, for example, are not accessible by the biomaterial 24.

In one embodiment, at this point of the treatment process, the foldable element(s) can be brought to the retracted position and the device 10 may then be moved away from the abscess cavity 20 or removed out of the patient after dispensing the biomaterial 24. In other embodiments, the device may be left at or around the abscess cavity 20 for about 7-10 days to remove any further predetermined material such as liquids released in the abscess cavity 20.

When it is determined, for example, by any imaging technique, that the predetermined materials are no longer accumulated in the abscess cavity 20, the securing means 14 can be moved to the retracted position within the catheter 12, and the device 10 can be retrieved, leaving behind the biomaterial 24 filled in the abscess cavity 20 (Fig. 5(5)).

The dispensing means 18 is adapted to provide the biodegradable and/or self-absorbable biomaterial 24 in the abscess cavity 20 of the anastomotic leak. The biomaterial 24 is self-absorbable such that the predetermined material in the abscess cavity 20 can be self-absorbed by the biomaterial 24. The biomaterial 24 is degradable and can remain in the abscess cavity 20 during the treatment of the anastomotic leakage and also during healing of the inflammation around the abscess cavity 20. As the biomaterial 24 is biodegradable and/or self-absorbable, there is no need for removal of the biomaterial from the patient during the treatment and also after the treatment is complete. Accordingly, the need for multiple and/or expensive endoscopic interventions in the conventional approach can be avoided and the patient's comfort level can be improved.

As shown in Fig. 5(6), the abscess cavity 20 can shrink over time, and the biomaterial 24 may degrade within the same time period, eventually healing and closing the fistula/abscess cavity.

In the above embodiment, it is described that the vacuum tube 16 is present in the catheter 12 when dispensing the biomaterial 24 with the dispensing tube 18. However, the invention is not limited thereto. In an alternative embodiment, the vacuum tube 16 can be removed from the device 10 after at least partially absorbing the predetermined material collected in the abscess cavity 20 and before inserting the dispensing tube 18 into the catheter to dispense the biomaterial 24 such that only one of the vacuum tube 16 or the dispensing tube 18 is present in the catheter 12 at a given time. Consequently, the vacuum tube 16 can be re-inserted into the catheter 12 when need arises, however, with or without the dispensing tube 18 being present in the catheter 12. The diameters of the catheter 12, the vacuum tube 16 and the dispensing tube 18 can be chosen such that either one or both of the vacuum tube 16 and the dispensing tube 18 are accommodated at a given time.

In the above embodiment, it is described that the vacuum tube 16 and the dispensing tube 18 are two separate tubes. However, the invention is not limited thereto. In an alternative embodiment, the device 10 could use the same means and/or the same tube 26 (having a diameter of D_{V/I}) for the purpose of absorbing the predetermined material collected in the abscess cavity 20 and for the purpose of dispensing the biomaterial into the cavity 20 (as illustrated in Fig. 6). This can particularly be advantageous in situations in which the catheter 12 has a diameter to accommodate only one of the vacuum tube 16 or the dispensing tube 18 at a given time (compared to Fig. 4). The diameter D_{V/I} is at least equal to the sum of the diameters Dᵥ (preferably about 2.4 mm - 5 mm) and D_{I} (preferably about 2.4 mm - 5 mm) and lower than the diameter D_{c} of the guiding catheter 12.

The above describes examples of various aspects of the invention and are not meant to be limiting.

## Claims

1. A device for treatment of an anastomotic leak comprising:
a catheter (12), wherein an end of the catheter (12) is configured to be positioned at a target site of the anastomotic leak, having predetermined material; and
a dispensing means (18), **characterized in that** the dispensing means (18) is configured to provide a biodegradable and/or self-absorbable biomaterial (24) in the target site such that the predetermined material is self-absorbed by the biomaterial (24).

2. The device of claim 1, further comprising a securing means (14) coupled to the end of the catheter (12),
wherein the securing means (14) is configured to be movable to an open position for securing the end of the catheter (12) to the target site.

3. The device of claim 1 or 2, further comprising an absorbing means (16) configured to absorb the predetermined material before and/or after the providing of the biomaterial in the target site.

4. The device of claim 2 or 3, wherein at least a part of the securing means (14) is within the catheter (12).

5. The device of claim 3 or 4, wherein at least a part of the absorbing means (16) is arranged within the catheter (12).

6. The device of any one of claims 1 to 5, wherein the absorbing means (16) comprises a tube provided with vacuum suction.

7. The device of any one of claims 1 to 6, wherein at least a part of the dispensing means (18) is arranged within the catheter (12).

8. The device of any one of claims 1 to 7, wherein the dispensing means (18) comprises a dispensing tube connected to a biomaterial supply.

9. The device of any one of claims 1 to 8, wherein the biomaterial (24) is either alone, combination, or derivative of any one or more of natural biodegradable polymeric biomaterial and/or synthetic biodegradable polymer.

10. The device of claim 9, wherein the natural biodegradable polymeric biomaterial is selected from any one of gelatin, collagen, fibrin, hyaluronic acid, chitin/chitosan, starch, silk, alginate and polyhydroxyalkanoate, and the synthetic biodegradable polymer is selected from any one of saturated aliphatic polyester such as poly(glycolic acid) (PGA), PLA, and PLGA copolymer, polyanhydride ,polyurethane, polyphosphazene.

11. The device of any one of claims 1 to 10, wherein the biomaterial (24) is configured to take the shape of the target site (20).

12. The device of any one of claims 1 to 11, wherein the biomaterial (24) is detectable with an imaging technique.

13. The device of any one of claims 1 to 12, wherein the biomaterial (24) is chargeable with antibiotics.

14. The device of any one of claims 1 to 13, wherein the biomaterial (24) is porous so as to allow the predetermined material to pass therethrough an/the absorbing/sucking means.

15. The device of any one of claims 1 to 14, wherein the biomaterial (24) is provided in a flowable form and configured to coagulate to a non-flowable form after a predetermined time.
